# EUROPEAN PATENT APPLICATION

(11) **EP 4 641 198 A1**
(43) Date of publication of application: **29.10.2025**
(21) Application number: 24382448.9
(22) Date of filing: 24.04.2024
(51) Int. Cl.: G01N 33/543

(54) **NANOPARTICLES COMPRISING A STARCH-IODINE COMPLEX AND THEIR USE FOR IMMUNODETECTION**

(71) Applicant: Universidad de Oviedo, 33003 Oviedo (ES)
(72) Inventor: BLANCO LÓPEZ, María del Carmen, 33006 Oviedo (ES); MATOS GONZÁLEZ, María, 33006 Oviedo (ES); GUTIÉRREZ CERVELLÓ, Gemma, 33006 Oviedo (ES); SAWERES ARGÜELLES, Clara, 33006 Oviedo (ES); SÁNCHEZ CALVO, Alberto, 33006 Oviedo (ES)
(74) Representative: Hoffmann Eitle

(57) **Abstract**

The invention relates to nanoparticles for immunodetection based on lateral flow assays, wherein the nanoparticles comprise an iodine-starch complex in the core of the nanoparticles, or on their surface. The invention furthermore relates to a composition comprising a plurality of such nanoparticles, an immunodetection sensor comprising the nanoparticles and an immunodetection method using them for visual and/or electrochemical detection.

## Description

### Technical field

The invention relates to nanoparticles for immunodetection based on lateral flow assays, wherein the nanoparticles comprise an iodine-starch complex in the core, or on their surface. The invention furthermore relates to a composition comprising a plurality of such nanoparticles, an immunodetection sensor comprising the nanoparticles and an immunodetection method using them for visual and/or electrochemical detection.

### Background of the invention

COVID-19 pandemic has highlighted the need for simple, affordable, and easy-to-use point-of-care devices that can be used in situ for the patient without specialized personal. Furthermore, such devices are of great interest for environmental control or food safety applications. Lateral flow immunoassays (LFIAs) have become one of the dominant options along the years. They are based on immunochromatographic membranes where the sample and the antibodies diffuse, and nano-labels are used to create a visual signal in order to confirm the presence or the absence of the analyte. Different types of nano-labels can be used, gold nanoparticles (AuNPs) being the most common ones. Gold nanoparticles have great catalytic and conductive properties with simple and well-known synthesis procedures.

Immunochromatographic assays are based on using a nitrocellulose chromatographic membrane immobilized with a monoclonal antibody on the test line (TL) and a secondary antibody on the control line (CL). The sample is added to the sample pad and the analyte binds to the labelled antibody on the conjugate pad. The sample then flows through the membrane by capillary action, where the target is captured by the TL antibody while the excess binds to the secondary antibody on the CL. The result is interpreted by comparing the appearance of the two lines in a visual way with measurement of optical signals as a quantitative method. However, this technique has obvious drawbacks such as low sensitivity or repeatability' , and the need to provide a quantitative result. Different alternatives are applied to improve the sensitivity of LFIAs such as other types of nanomaterials or the use of other detection techniques instead of optical analysis with better sensitivity such as fluorescence or magnetic nanoparticles, but these require complex and bulky readers. There remains thus a need for providing methods for enhancing the signal in LFIAs to overcome the above disadvantages and to allow the use of portable and small devices for the signal readout.

Electrochemistry can be applied to the LFIAs because of the catalytic and conductive or electroactive properties of gold nanoparticles (AuNPs). This technique can improve the sensitivity of these tests in a cheap, simple, and rapid analysis in a coupling with an electrochemical cell. This involves applying or inserting screen-printed electrodes into the strip and by using an electrochemical label, measuring a current signal related to the analyte. In this way, the POC device minimize matrix effects increasing the sensitivity.

Different labels can be used in electrochemical LFIAs (eLFIAs). There is research of eLFIAs based on enzymatic tracers² or quantum dots (QDs)³. However, gold nanoparticles which can be the most common label for visual analysis in lateral flow immunoassays, are rarely used for this purpose. In some of these cases they measure both the horseradish peroxidase (HRP) enzyme signal as an amplification probe⁴ and dissolved Au(lll) ions by square wave voltammetry⁵, while in others they perform amperometric measurements of the analyte itself ⁶.

Gold nanoparticles need to be conjugated to antibodies. This process is frequently performed by passive adsorption taking advantage of the great affinity of sulfur atoms or thiol groups on the aminoacid chains, for the gold surfaces. Then, once the antibodies are already adsorbed on the surface of the nanoparticle, the remaining free sites are blocked with proteins such as bovine serum albumin, caseine, or polymers such as polyethylene glycol⁷ to avoid unspecific adsorptions.

Biodegradable nanoparticles have attracted increasing interest in recent years for different purposes. Starch nanoparticles (SNPs) are natural, abundant, renewable and inexpensive to produce⁸. SNPs provide different physicochemical and biological properties compared to native starch. Among their advantages are higher solubility, reaction surface, absorptive capacity and biological penetration rate. The use of SNPs in various applications, such as the food industry, production of nanocomposite materials, for biomedical applications packaging, in cosmetics and as colloidal stabilizers has been described.

Iodine can be measured electrochemically through coulometry⁹, a technique where the potential of the working electrode is kept constant, allowing the analyte to exchange electrons across the electrode-solution interface. Various types of iodine nanoparticles and their use in different fields are disclosed in the prior art. Iodine nanoparticles and their use as X-ray reagents have been described for example in US 2021/0069352 A1, where the iodine nanoparticles are a reaction product of functionalized triiodobenzene, linking monomers, and biocompatible polymers. Nanoparticles formed from iodide and ions of an alkali metal or alkaline earth metal and their use for radiation therapy has also been described (WO2023/039415 A2). However, the use of such iodine-carrying nanoparticles in LFIAs has not been described to date.

The inventors have set out to provide new nanoparticles that are suitable for use in LFIAs that can overcome the disadvantages of the detection methods of the prior art, such as low sensitivity and repeatability, the requirement of complex and large readers when using fluorescence or magnetic nanoparticles instead of optical analysis to enhance the signal, and the fact that two detection methods can be used in the same system i.e. optical or electrochemical detection depending on the LFIA chosen.

### Summary of the Invention

The present invention therefore relates to a nanoparticle for immunodetection based on lateral flow assays, wherein the nanoparticle comprises an iodine-starch complex.

In a first aspect of the nanoparticles of present invention the nanoparticle has a core and a shell and the iodine-starch complex is located in the core of the nanoparticle.

In one embodiment the core of the nanoparticle comprises between about 1% - 4% (w/v), preferably between about 1% - 2% (w/v) of the iodine-starch complex.

In one embodiment of this aspect the shell comprises at least one polyelectrolyte, at least one surface active agent and at least one stabilizer.

In one embodiment the polyelectrolyte is selected from the group consisting of polylactic-co-glycolic acid (PLGA), Polyethylene Glycol (PEG), polylactic acid (PLA), poly-ε-caprolactone (PCL), or combinations thereof.

In one embodiment the surface-active agent is selected from the group consisting of phospholipids or non-ionic surfactants, or a combination thereof.

In one embodiment the phospholipid is phosphatidylcholine or phosphatidylserine or a combination thereof.

In one embodiment the non-ionic surfactant is Span 60 or Span 80, or combinations thereof.

In one embodiment the at least one stabiliser is selected from the group consisting of polyvinylalcohol, dodecanol, cholesterol, cholesteryl hemi succinate, or combinations thereof.

In one embodiment the mean diameter of the nanoparticle is between about 150 nm to about 250 nm, preferably between about 190 nm to about 210 nm.

In one embodiment the nanoparticle is conjugated with a protein or antibody.

In one embodiment the mean diameter of the nanoparticle conjugated with a protein or antibody is between about 200 nm to about 300 nm, preferably between about 250 nm to 280 nm.

In a second aspect of the nanoparticles of present invention the iodine-starch complex is located on the surface of the shell of the nanoparticle.

In one preferred embodiment of this second aspect the nanoparticle is a gold nanoparticle (AuNP).

In one embodiment the nanoparticle have been blocked in a 0.1% starch (w/v) starch-iodine solution.

In one embodiment the mean diameter of the AuNP without the iodine starch complex on the surface is between about 20 nm - 50 nm, preferably between about 30 nm - 40 nm.

In one embodiment the mean diameter of the AuNP with the iodine starch complex on the surface is between about 60 nm to 90 nm, preferably between about 70 nm to 80 nm.

In a third aspect the present invention relates to a composition comprising a plurality of nanoparticles as described herein.

In one embodiment of this aspect the composition comprises between 1 - 4% (w/v) of native quinoa starch nanoparticles previously stained with Lugol's reagent (containing 5% (w/v) of iodine and 10% (w/v) of potassium iodide). In a preferred embodiment the nanoparticles have been stained with a volume ratio of nanoparticle to Lugol's reagent of 1:2.

In a fourth aspect the present invention relates to an immunodetection sensor comprising the nanoparticle for immunodetection based on lateral flow assay or the composition comprising a plurality of nanoparticles as described herein.

In a fifth aspect the present invention relates to an immunodetection method comprising the steps of
(i) mixing a sample with the nanoparticles or the composition comprising a plurality of nanoparticles as described herein, wherein the nanoparticle(s) have been conjugated to an antibody or protein for detection of an analyte in a sample; and
(ii) detecting an electrochemical signal, an optical signal, or both.

### BRIEF DESCRIPTION OF THE FIGURES

Figure 1: Hydrodynamic diameter distribution profile of SNPs stained with iodine.
Figure 2: TEM images taken at 100000x showing SNPs and agglomerates with a range of sizes from 50 nm up to 500 nm.
Figure 3: FTIR spectra of the SNPs before (dashed line) and after (solid line) staining them with iodine.
Figure 4: Scheme showing the synthesis and structure of the SNPs in PLGA-PVA/PC by emulsion/solvent evaporation method.
Figure 5: TEM micrograph of the lipid polymer hybrid nanoparticles encapsulating stained SNPs.
Figure 6: Hydrodynamic size distribution profiles of SNPs in PLGA-PVA/PC before (dotted line) and after (solid line) conjugation with a neutravidin concentration of 1 mg/mL.
Figure 7: Photography (front view) and schematic illustration of the biotin-neutravidin affinity test (side view).
Figure 8: Coulometry performed at a fixed potential of + 0.75 V measuring signal at the test line in the CN95 membranes (dotted line for negative control and dashed line for positive TL) and FF120HP (long dash for negative control and solid line for positive TL).

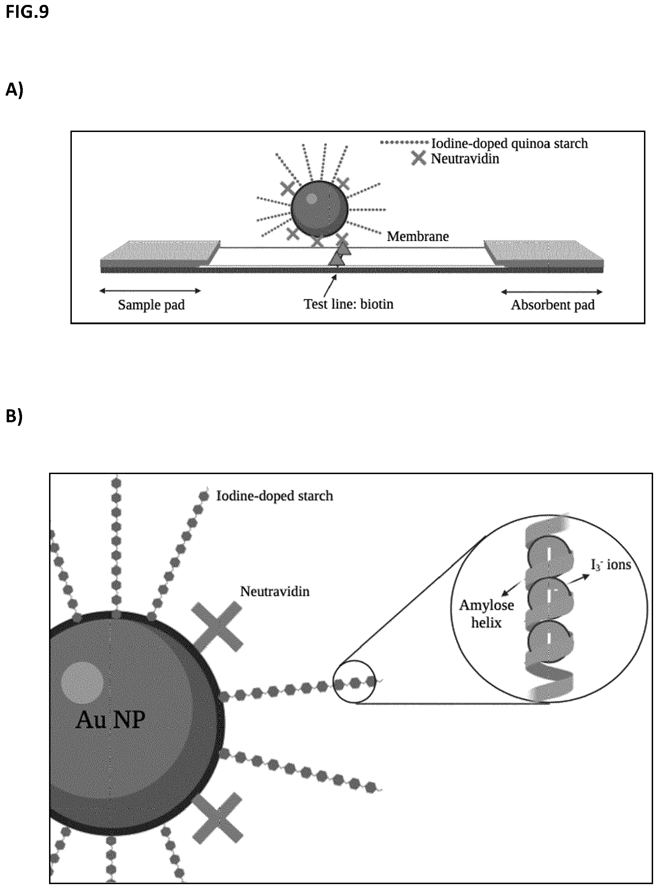

Figure 9: A) Schematic illustration of the biotin-neutravidin affinity assay (side view) based on Au NPs as reporter labels blocked with iodine-doped quinoa starch. B) Scheme of Au NP blockage. Enlarged is the schematic of the starch-iodine complex, in which the I₃⁻ ions are embedded in the amylose helix that forms starch together with amylopectin.
Figure 10: Hydrodynamic size distribution profiles of gold NPs before (dotted) and after conjugation with different blocking compounds: BSA (dashed) and starch-iodine (solid).
Figure 11: Normalized absorbance UV-VIS spectra of colloidal gold (dotted) and the neutravidin conjugates blocked with BSA (dashed) and iodine-doped starch (solid). The maximum wavelength shifts towards red in the case of the conjugates.
Figure 12: Coulometry of lateral flow electrochemical assay: negative control (dotted), 0.15 mg/mL neutravidin (dashed), and 0.3 mg/mL neutravidin (solid) blocked with BSA 1 mg/mL (E = 0.5V, t = 100 s).
Figure 13: Coulometry of lateral flow electrochemical assay: negative control (dotted), 0.15 mg/mL neutravidin (dashed), and 0.3 mg/mL neutravidin (longdash) blocked with iodine-doped starch 1 mg/mL (E = 0.5V t = 90 s).

### Detailed description

The present invention may be understood more readily by reference to the following detailed description of the preferred embodiments of the invention, and to the examples included therein.

As described in more detail above there is an unmet need for improved LFIAs with increased sensitivity, specificity, versatility and simplicity of use.

The inventors have set out to provide new nanoparticles that are suitable and beneficial for use in LFIAs that can overcome the disadvantages of the detection methods of the prior art, such as the low sensitivity and repeatability, the requirement of complex and large readers when using fluorescence or magnetic nanoparticles instead of optical analysis to enhance the signal and the fact that two detection methods can be used, i.e. only optical visualization only electrochemical detection depending on the LFIA chosen, or both types of detection on a single test.

The inventors have therefore developed nanoparticles that combine an enhanced signal, versatility in the method of signal detection depending on the needs and ease of use.

The present invention therefore relates to a nanoparticle for immunodetection based on lateral flow assay, wherein the nanoparticle comprises an iodine-starch complex.

As is described in more detail here below the inventors have found that the nanoparticles can be formed by encapsulating the iodine-starch complex in a micelle, also referred to as a shell herein. On the other hand, the iodine-starch complex can be doped onto the surface of a nanoparticle, such as for example a gold nanoparticle (AuNP).

### NANOPARTICLES WITH ENCAPSULATED IODINE-STARCH COMPLEX

As described in more detail in examples 1 and 2 starch nanoparticles (SNPs) with longer chain amylose have been selected due to the strong blue color they obtain when iodine and the amylose form a complex and due to the stability of this complex. The iodine-stained SNPs were then encapsulated using a single emulsion/solvent method and hybrid nanoparticles, i.e., iodine-stained SNPs encapsulated in PLGA-PVA/PC were prepared.

In one embodiment of present invention the starch is selected among the sources with longer chain amylose. Starch derived from quinoa is preferred.

In one embodiment of the present invention the nanoparticle therefore has a core and a shell and the iodine-starch complex is located in the core of the nanoparticle. In one embodiment the core of the nanoparticle comprises between about 1% - 4% (w/v), preferably between about 1% - 2% (w/v) of iodine-starch complex.

The shell of the nanoparticles can be further functionalized to bioconjugate it to the desired protein or antibody for detection. In addition, the shell allows to have more nanoparticles attached to a single antibody. This improves the sensitivity of the detection, versus to a single particle attached to a single antibody.

The shell of the nanoparticles of present invention can comprise a polyelectrolyte that can bind the cargo, such as the starch iodine complex, surface active agents acting as main membrane components and stabilizers that provide the required stability to the micelles.

Encapsulation by Lipid-polymer hybrid nanoparticles (LPHNPs) has been previously disclosed¹⁰. Encapsulation by LPHNPs offers several advantages such as enhancing device sensitivity as well as the possibility to modify the properties of the surface to provide a better interaction with biological molecules.

In one embodiment of present invention the polyelectrolyte is selected from the group consisting of polylactic-co-glycolic acid (PLGA), Polyethylene Glycol (PEG), polylactic acid (PLA), poly-ε-caprolactone (PCL), or combinations thereof.

Poly (lactic-co-glycolic acid) (PLGA) is described in the prior art as an alternative to other conventional nanocolloids, such as nanoemulsions or liposomes that allows the possibility to modify surface properties to provide stealthiness and/or better interaction with biological materials¹⁰. In a preferred embodiment the polyelectrolyte is therefore polylactic-co-glycolic acid (PLGA).

In one embodiment the surface-active agent is a phospholipid, such as for example phosphatidylcholine, or phosphatidylserine. In one embodiment the surface-active agent is a non-ionic surfactant, such as for example Span 60 or Span 80. Two or more surface-active agents can also be combined.

The preferred coating is phosphatidylcholine. Phosphatidylcholine (PC) is a neutral phospholipid that adsorbs and self-assembles onto the surface of the hydrophobic polymer through hydrophobic interactions with the goal of reducing the free energy of the system.

In one embodiment the at least one stabiliser is selected from the group consisting of polyvinylalcohol, dodecanol, cholesterol, cholesteryl hemi succinate, or combinations thereof. Cholesteryl hemisuccinate is preferred because it provides chemical groups for covalent binding to the detection antibody used in the immunoassay.

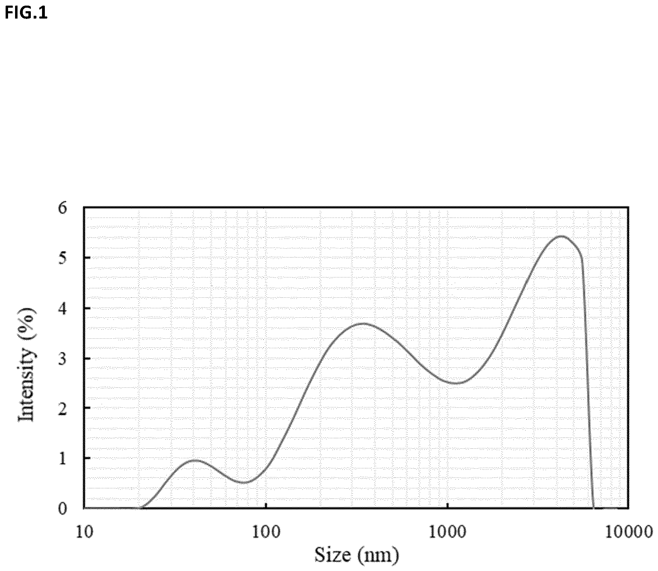

As described in Example 3 and shown in Figure 1 the diameter of the SNPs before iodine staining was about 54 to about 80 nm. The iodine-stained SNPs were shown to have a diameter of between about 400 nm nm to about 500 nm, meaning that the micelles with the encapsulated stained SNPs agglomerated. Aggregation is not wanted, as it impedes the flow through the membrane. However, since the analytical signal for detection is proportional to the number of nanoparticles used, it is necessary to use a plurality of nanoparticles. Therefore, a compromise between the degree of agglomeration (controlled) and maximum signal desired has to be achieved.

Furthermore, the size of the nanoparticles is important to allow the particle-antibody aggregates to flow through the pores of the membrane of a lateral flow immunoassay. The membranes are defined by their flow rate. Specifically suitable membranes for putting the nanoparticles of present invention into practice in a LFIA have a flow rate of between 75s/4cm to 120s/4cm. Especially suitable membranes are for example Sartorius CN95 (flow rate of 95s/4cm), Cytiva FF120HP (flow rate of 120 s/4cm), Millipore HiFlow Plus HFC07504 (flow rate of 75s/4cm).

The nanoparticles of present invention comprise in one embodiment the mean diameter of the final core-shell nanoparticle of between about 150 nm to about 250 nm, preferably between about 190 nm to about210 nm. In the most preferred embodiment, the mean diameter is about 193 nm to about 200 nm. It is to be understood that this size is without conjugated antibody or protein to the nanoparticles. Once the protein or antibody is conjugated to the nanoparticle, the conjugated nanoparticle has a mean diameter of between about 200 nm to about 300 nm, preferably between about 250 nm to 280 nm. In the most preferred embodiment, the mean diameter is about 255 nm to about 271 nm.

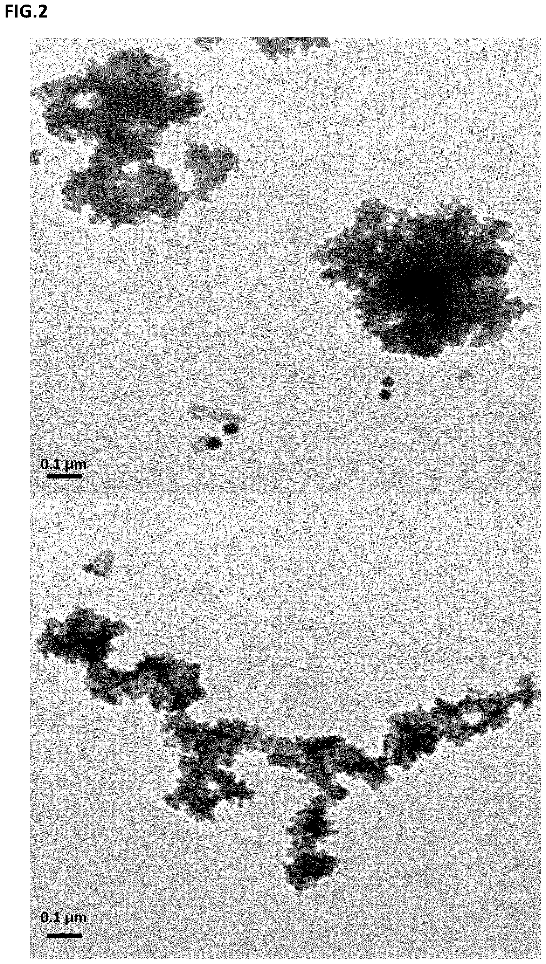

The particle morphology and the aggregation structure of the SNPs were determined by TEM and the agglomeration of the nanoparticles was confirmed with TEM images in which both individual nanoparticles and agglomerates of several different sizes were observed (Figure 2).

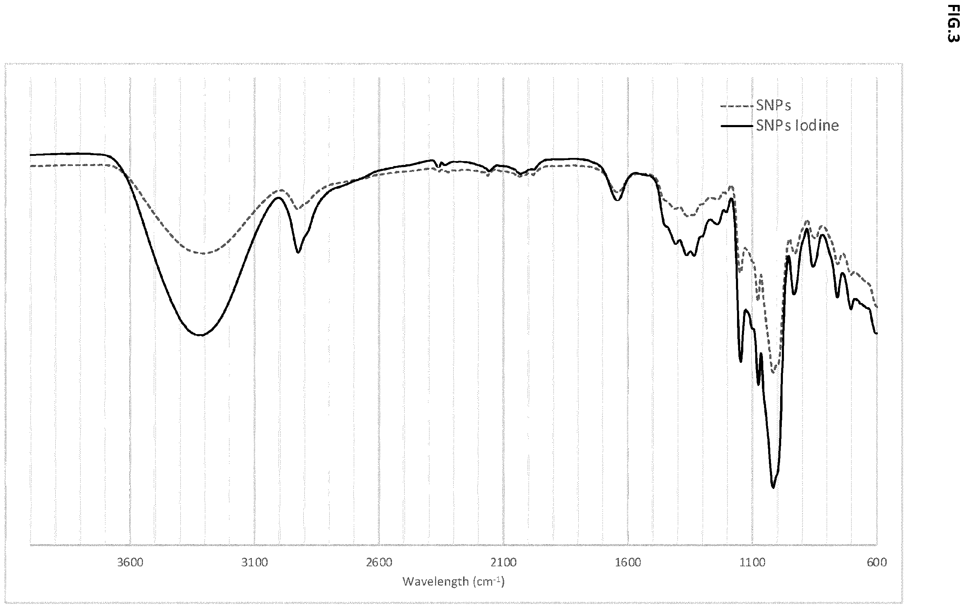

In one embodiment the present invention therefore relates to a composition comprising a plurality of nanoparticles as described herein, wherein the nanoparticles form agglomerates. Fourier transform infrared spectroscopy (FTIR) was used to determine the molecular structure of the iodine-stained SNPs and starch nanoparticles and to verify possible molecular structure alterations. The spectra of both the SNPs and the SNPs stained with iodine showed exactly the same bands, confirming that the complex amylose-iodine was created through secondary chemical interactions (Figure 3).

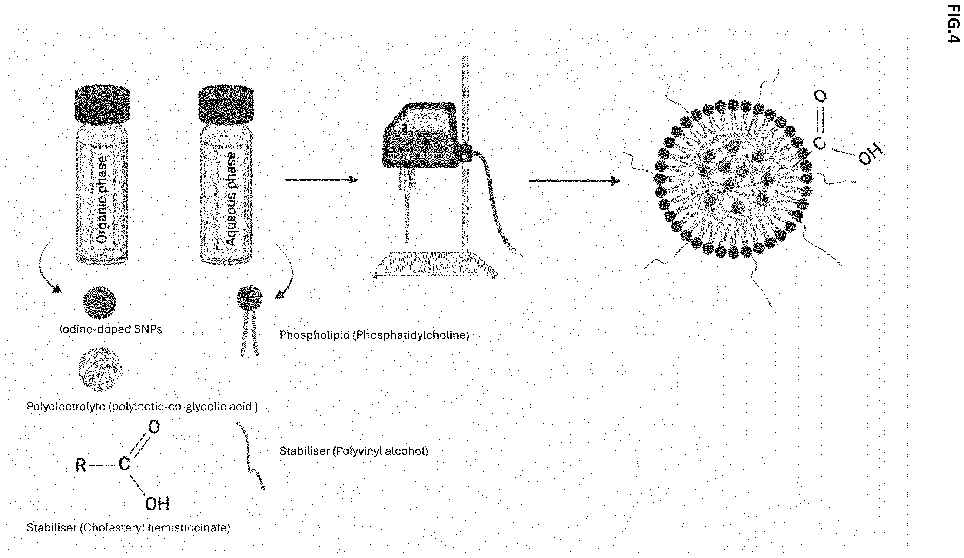

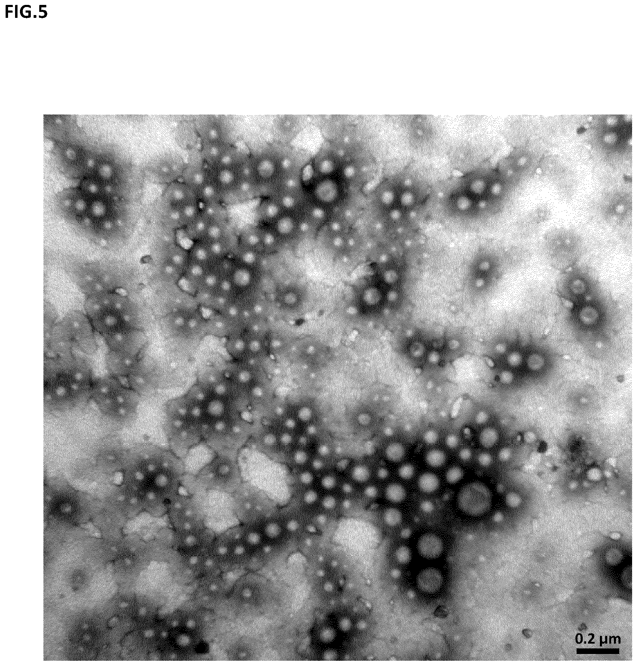

As described in Example 4, lipid-polymer hybrid nanoparticles encapsulating iodine-stained SNPs were synthesized by emulsion/solvent evaporation method. In figure 4, the scheme of the synthesis of the nanocapsule is depicted. The formulation consisted of an organic phase containing PLGA acting as a carrier entrapping the SNP transferred to this phase and cholesteryl hemisuccinate to functionalize the hybrid nanoparticle. On the other hand, the aqueous phase contained phosphatidylcholine as the lipid layer coating and poly-vinyl alcohol, a non-ionic surfactant, as a stabilizer. The encapsulation was confirmed by TEM, shown in figure 5, where it can be seen that the final core-shell nanoparticles had a spherical shape. Whilst different shapes of nanoparticles have been used in lateral flow assays, such as for example nanorods or nanoflowers, the spherical shape provides the advantage of having a larger interfacial area being available. The spherical shape thus provides more surface for functionalization and detection.

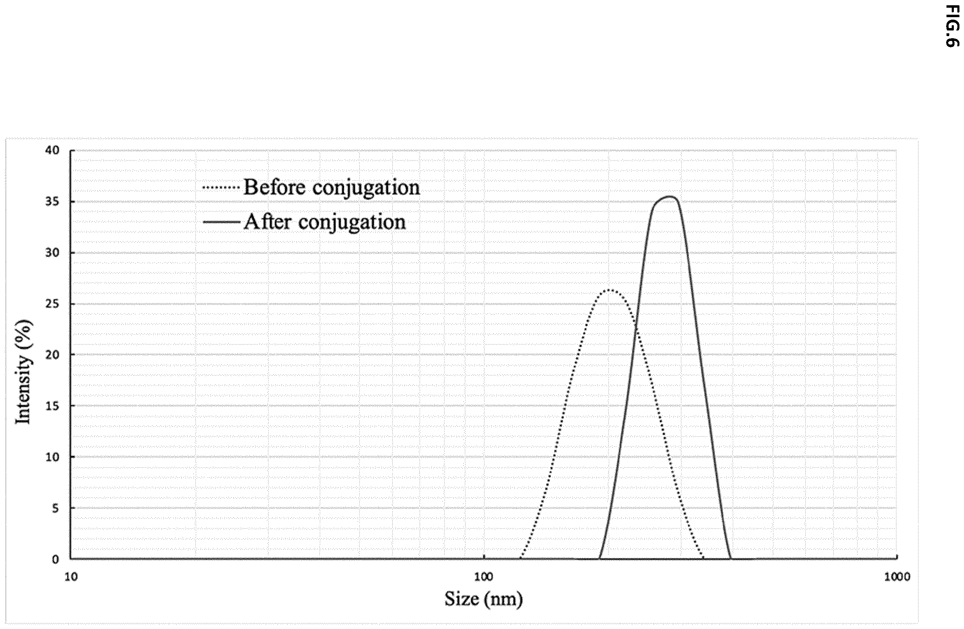

Bioconjugation of lipid-polymer encapsulated iodine-stained SNPs was tested with Neutravidin (Example 5). The average hydrodynamic size of the SNPs was measured with DLS (figure 6) before and after the conjugation, showing an increase of the size confirming a successful conjugation.

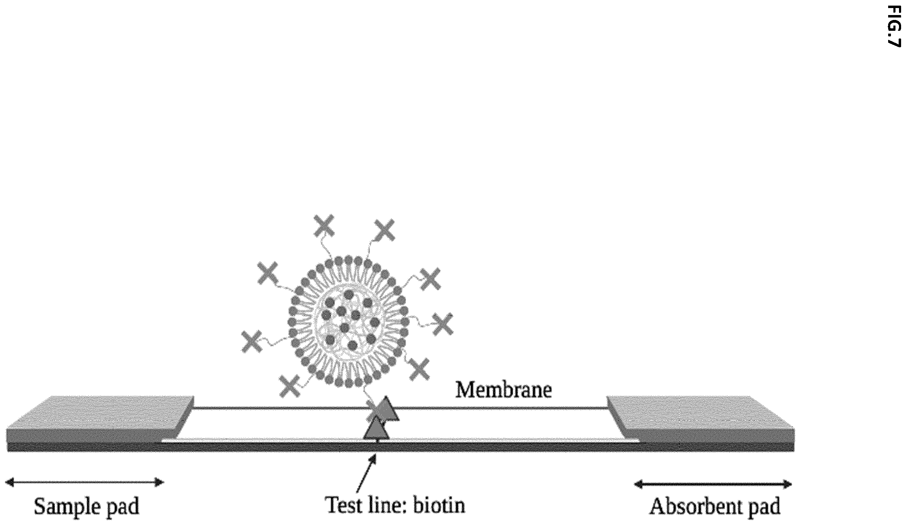

Finally, the use of the SNPs was tested in LFIAs based on the reaction between biotin and neutravidin as further detailed in Example 6. The color intensity of the test line was quantified by reflectance measurements by using a portable strip reader and the signal obtained was compared to another standard assay based also on Neutravidin/biotin LFIA but using 40 nm colloidal gold-neutravidin conjugates as a visual label. Only the neutravidin coated nanoparticles were retained on the biotin-BSA test line. A simplified schematic representation of the biotin-neutravidin interaction in LFIA is shown in Figure 7.

The dried test line was analyzed by reflectance measurements and the results of the reflectance measurements for gold and hybrid nanoparticles were compared in terms of intensity (mV), with the density of the latter being slightly higher for a similar concentration of neutravidin, making this label an advantageous alternative to the gold nanoparticles (see table 2 in Example 6).

To assess the suitability for electrochemical measurement of the signal, the iodine and triiodide present in the starch nanoparticles in the test line was measured through coulometry.

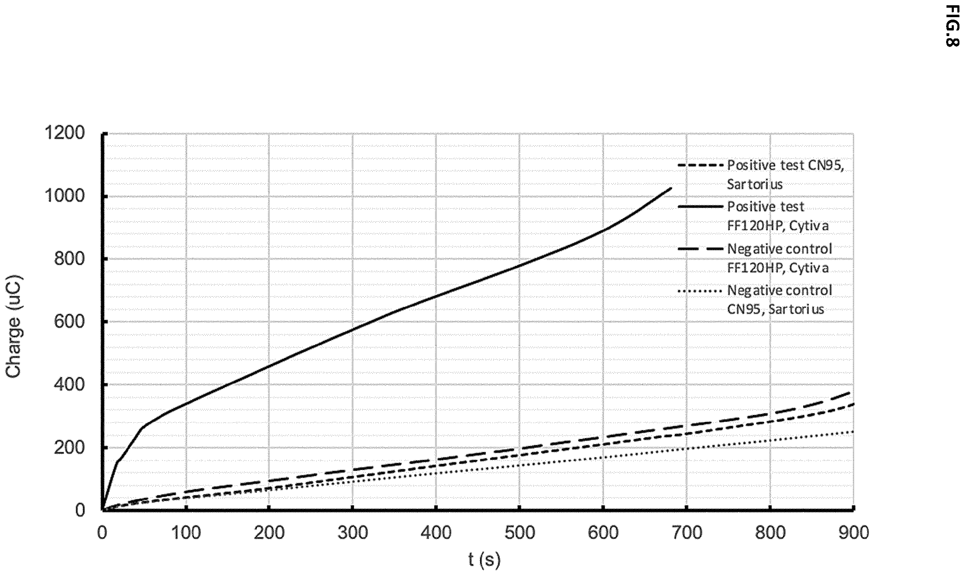

Results showed an increasing of charge in presence of nanoparticles in both types of membranes as it can be seen in Figure 8.

In summary, the inventors could show that the iodine-stained SNPs are suitable for visual and electrochemical detection and can thus provide for a sensitive, versatile and simple method for detection.

### NANOPARTICLES WITH IODINE-STARCH COMPLEX ON SURFACE

In a second aspect of present invention iodine-starch complex as described above is located on the surface of the nanoparticle.

In one preferred embodiment of this second aspect the nanoparticle is a gold nanoparticle (AuNP).

As described in more detail in Example 7 gold particles (AuNPs) were bio-conjugated with neutravidin and subsequently iodine-stained starch molecules were attached to the surface of the AuNPs.

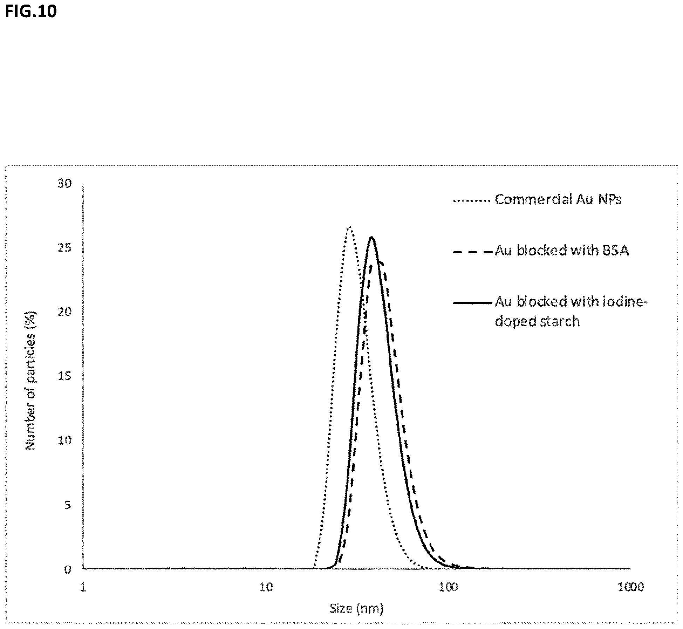

The hydrodynamic size and the zeta-potential of the AuNP conjugates blocked with starch and BSA was determined by DLS measurements and were carried out to confirm bioconjugation by comparing the size of the hydrodynamic radius before and after the conjugation reaction (Fig. 10). As can be seen in table 3 the bare commercial AuNPs were smaller than both conjugates showing that the conjugation process was successful in both cases.

In one embodiment of the AuNPs of present invention the mean diameter of the nanoparticle is between about 20 nm - 50 nm, preferably between about 30 nm - 40 nm. In this size they provide for maximum intensity of their colour and can thus be visualized very well.

After conjugation with a protein or antibody (conjugated nanoparticles) they have a final diameter of between about 60 nm to about 90 nm, preferably about 70 nm to about 80 nm. In a preferred embodiment the mean diameter is between about 74 nm to 76 nm.

The nanoparticles are first conjugated with the protein or antibody and then the iodine starch complex is added as blocking agent into the free spaces left on the surface of the nanoparticle between the conjugated antibodies. The inventors have found that by using these spaces between the conjugated proteins/antibodies the iodine starch provides for a double benefit. On the one hand the filling of the spaces reduces unspecific adsorption and on the other hand the particles at the test line can be better visualized.

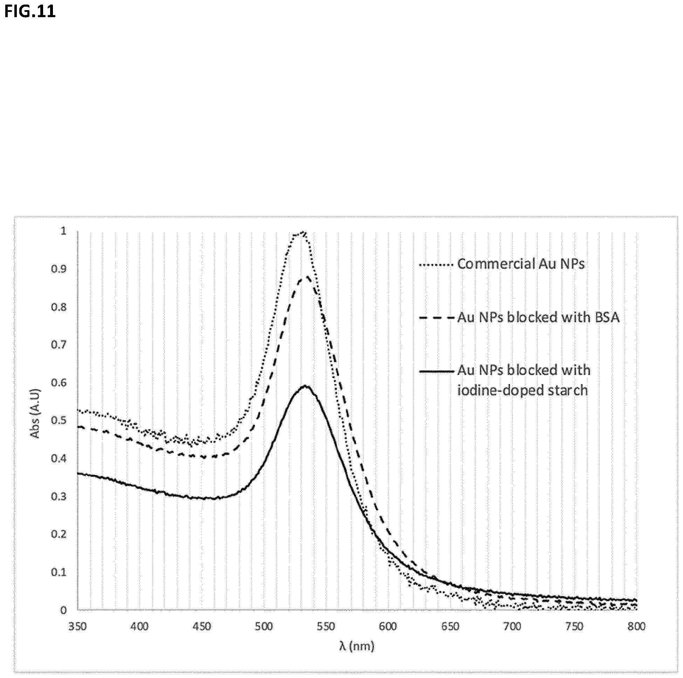

The ultraviolet-visible spectrum was also measured to verify both conjugation and agglomeration of the nanoparticles. The absorbance measurements of gold nanoparticles were employed to track the adsorption of proteins and antibodies on their surface. A strong absorbance peak between 460-560 nm was observed due to the surface plasmon resonance (SPR) of colloidal gold, and its maximum wavelength (λmax) varied depending on the size of the nanoparticles. The UV-Vis absorbance spectra of both the bare nanoparticles and the conjugates were measured between 350 nm and 800 nm (Fig. 11).

The maximum wavelength of AuNPs was 530 nm. A red-shift to a λmax of 532 nm and 534 nm was observed when the conjugation reaction was performed, blocking with BSA and starch-iodine, respectively. This is an indication of a change in the refractive index of the nanoparticles due to the neutravidin layer on the surface of the nanoparticles.

The inventors could therefore show that the conjugated gold nanoparticles were stable and could maintain the desired color, which also indicates that no aggregation occurred when blocked with starch. The developed nanoparticles are therefore suitable for the application in immunodetection as envisaged herein.

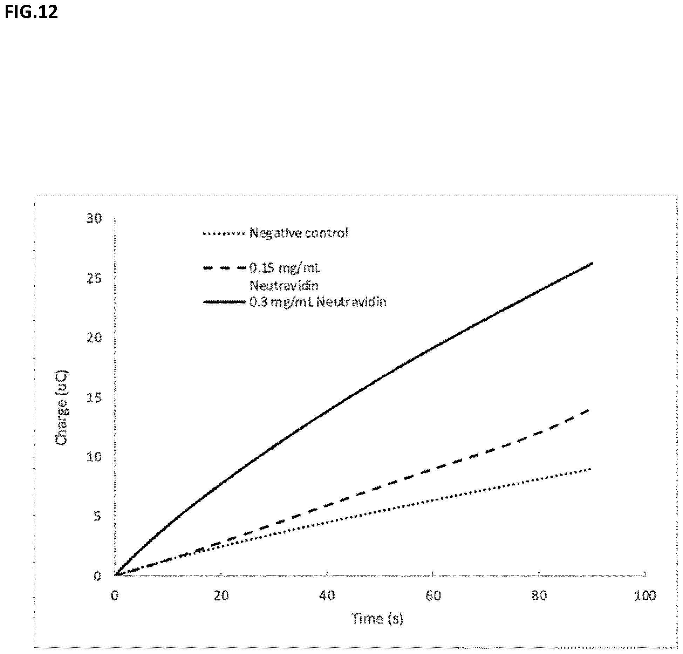

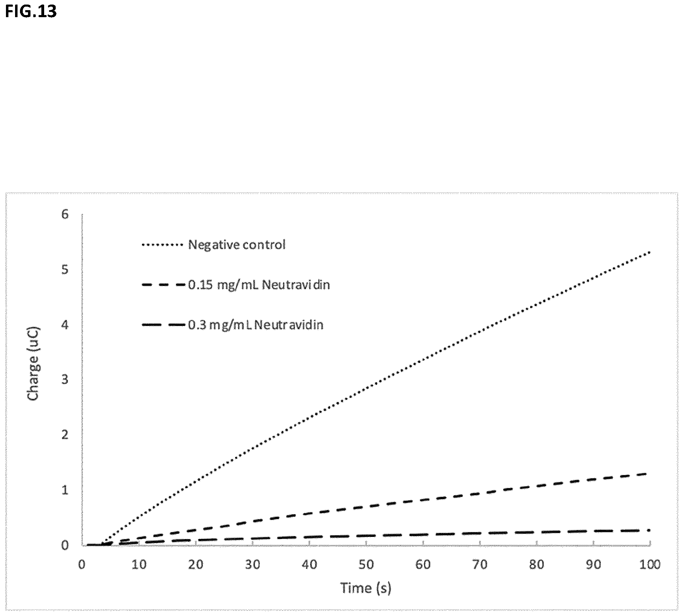

Iodine-doped starch-blocked gold nanoparticles were tested as labels in LFIAs based on the biotin-neutravidin affinity assay as described in Example 7 and electrochemical measurement was performed. Figure 12 shows the results of the coulometry for conjugates blocked with Bovine Serum Albumin (BSA). No significant trend in the measurements was observed as the analytical signal of the negative test is higher than the positive ones, which do not follow any trend either. However, in the case of the conjugates blocked with iodine-doped starch (Fig.13), a correlation between the electrochemical and visual signals was found. Both signals increase as the amount of conjugated neutravidin added increases. Therefore, with this procedure electrochemical measurements can be performed and used for quantification in LFIAs. This overcomes one of the actual limitations for simple and portable quantification of LFIAs.

The inventors have surprisingly found that when starch was used to modify the gold nanoparticle surface after the conjugation unspecific adsorption was not observed, indicating that starch is a very efficient blocking agent. When it is combined with iodine, it acts also as carrier for the electrochemical probe that generates the electroanalytical signal.

In summary, the inventors have found that iodine-stained starch polymers can be used as a label and/or blocking agent in immunoassays, specifically in combination with AuNPs. Some of the benefits of using such iodine-stained starch polymers labels or blocking agent is that they are renewable, biodegradable, biocompatible and cheap to produce¹¹. The fact that the starch materials develop blue color through the interaction with iodine and that iodine can be measured electrochemically through coulometry opens a versatile detection system that can be used depending on its needs^{12,13}.

In a fourth aspect the present invention relates to an immunodetection sensor comprising the nanoparticle for immunodetection based on lateral flow assay or the composition comprising a plurality of nanoparticles as described herein.

In a fifth aspect the present invention relates to an immunodetection method comprising the steps of
(i) mixing a sample with the nanoparticles or the composition comprising a plurality of nanoparticles as described herein; and
(ii) detecting an electrochemical signal, an optical signal, or both.

In this aspect the nanoparticles are conjugated nanoparticles. This means that the nanoparticles have been modified to contain an antibody or protein for detection bound to their surface which will detect the desired analyte in the sample.

### EXAMPLES

### Example 1: SNPs (Starch nanoparticles) staining with iodine

Native starch from both quinoa and amaranth were dissolved separately with a concentration of 20 mg/mL in milliQ water. Then, a solution of Lugol's reagent (5% I2, 10% KI) was added to native starch solution at a volume ratio of 1:2 followed by a reaction time of 30 min at stirring conditions of 24 rpm. Nanoparticles obtained were precipitated with absolute ethanol and centrifuged at 9500 rpm for 5 minutes. Afterwards, two washes with milliQ water and ethanol were performed to finally resuspend the nanoparticles in milliQ water.

Different trends were observed after adding the Lugol's solution to the colorless starch solutions of both quinoa and amaranth. A reddish like solution was observed in case of amaranth starch, whilst, in case of quinoa starch, the solution instantly became dark blue. This can be explained based on the foundations of the starch reaction with the triiodide ion (10). Starch is composed by two glucose polymers: amylopectin, which is the major one (around 75%) and amylose (around 25%). The molecular iodine and the triiodide ion react to amylose through secondary chemical interactions (rearranging with dipole moments parallel to the axis of the amylose helix). Depending on the chain length of the amylose, in the case of short chains, the amylose-iodine complex can have a purple-brown color, which would be the case of the amaranth solution, as both SNPs from amaranth and quinoa are reported to have a similar percentage of amylose (20.90% and 20.95% respectively) (11). It is known that only the blue complex between the triiodide and the iodine with the amylose is stable whereas the complex of the iodine species with the amylopectin (red) is less stable. Hence, quinoa SNPs were selected due to their strong blue color, making them suitable for this assay.

### Example 2: Synthesis of hybrid nanoparticles encapsulating SNPs-iodine

Iodine-stained SNPs were encapsulated using a single emulsion/solvent method. An aqueous phase was prepared containing 10 mg phosphatidylcholine, as the sole membrane component, and 1% polyvinyl alcohol (w/v) in milliQ water, whilst an organic phase, consisting of a 12.5% (v/v) solution of methanol in chloroform, containing 30 mg of poly (lactic-co-glycolic acid) (PLGA) and 1% (w/v) cholesteryl hemisuccinate of the total membrane compounds (PC).

The preparation of the hybrid nanoparticles (SNPs-iodine in PLGA-PVA/PC) was done following the next protocol: 2 mL of organic phase, containing 1 mL of dispersed SNPs-iodine, were emulsified in 6 mL of the aqueous phase, obtaining an oil-in-water (O/W) emulsion under continuous sonication at 55% amplitude for 2 min in an ice bath. The emulsion was then placed on a mechanical stirrer (witeg Labortechnik GmbH, Germany) with a magnet to evaporate the organic solvent and form the particles overnight. Hybrid nanoparticles obtained were purified by size exclusion chromatography (SEC) with a solution of SepharoseTM CL-4B in milli-Q water 67.5% (v/v).

### Example 3: Characterization of the SNPs-I and LPHNPs

### 3.1. Particle size distribution

Hydrodynamic size and homogeneity of the SNPs-iodine particles were obtained by dynamic light scattering (DLS) using a Zetasizer Nano ZS instrument (Malvern Instruments Ltd., Malvern, UK). Samples were measured with the 173° backscatter detector in low volume disposable cuvettes (Malvern Instruments Ltd., Malvern, UK). The average diameter was 434 ± 34 nm with three different populations of nanoparticles (Figure 1). Size of nanoparticles before staining them was 68 ± 13 nm, suggesting an agglomeration of the nanoparticles. A great polydispersity was also observed as a PDI value of 1.00 was obtained.

### 3.2. Particle morphology

Particle morphology, and the aggregation structure of the SNPs was determined by TEM. An aliquot of an aqueous suspension of the samples was placed on a transparent carbon sheet with copper grid support and analyzed with MET JEOL-2000 EXII TEM (Saint-Herblain, France). The agglomeration of the nanoparticles could be confirmed with TEM images (Figure 2) in which both individual nanoparticles and agglomerates of several different sizes were observed.

### 3.3. Molecular structure

Fourier transform infrared spectroscopy (FTIR) was used to determine the molecular structure of the iodine-stained SNPs and starch nanoparticles and to verify possible molecular structure alterations. FTIR spectra were recorded in a Fourier transform infrared spectrophotometer (Varian 620-IR, Thermo Fisher Scientific Inc., U.S.A.) at room temperature. Dried powder samples of approximately 1 mg were measured directly and spectra were recorded between 650-4000 cm⁻¹ (medium infrared band).

The spectra of both the SNPs and the SNPs stained with iodine showed exactly the same bands, lacking any halogen bonding band, therefore confirming that the complex amylose-iodine was created through secondary chemical interactions (Figure 3). In both cases, the hydroxyl groups appeared at a wavelength around 3300 cm⁻¹. At a wavelength of 2900 cm⁻¹, the characteristic band corresponding to C-H stretching vibration of the glucose unit12 was observed. The peak around 1600 cm-1 may correspond to the bending vibration of the H-O-H group of starch-bound water (13).

### Example 4: Synthesis and characterization of the lipid-polymer hybrid nanoparticles encapsulating SNPs-iodine

Then lipid-polymer hybrid nanoparticles encapsulating iodine-stained SNPs were synthesized by emulsion/solvent evaporation method. In figure 4, the scheme of the synthesis of the nanocapsule is depicted. The formulation consisted of an organic phase containing PLGA acting as a carrier entrapping the SNP transferred to this phase and cholesteryl hemisuccinate to functionalize the hybrid nanoparticle. On the other hand, the aqueous phase contained phosphatidylcholine as the lipid layer coating and poly-vinyl alcohol, a non-ionic surfactant, as a stabilizer.

The encapsulation was checked by TEM, shown in figure 5, where it can be seen that the nanoparticles had a spherical shape.

### Example 5: Bioconjugation of lipid-polymer SNPs-Iodine

A fresh solution 0.097 M of EDC and 0.261 M of NHS in MES buffer pH 5.5 was prepared. 100 µL of this solution was added to 1 mL of the lipid-polymer SNPs-I. In order to activate the carboxylic groups of the nanocapsule, it was allowed to react for 15 minutes under shaking. Then, it was centrifuged at 5000 × g for 4 minutes and washed with 200 µL of MES buffer, followed by an addition of 200 µl of a 0.5 mg/mL Neutravidin solution in MES pH 7.4 and left shaking for 3 h. After that, another shaking was done. After this time, another centrifugation at 5000 x g for 4 minutes was done followed by another washing step with MES buffer.

After the activation of carboxylic groups, a blocking step of un unreacted carboxylic groups unbound with neutravidin was done. 100 µL solution of CH₃O-PEG-NH₂ (5mg/mL) was added followed by a reaction time of 20 min. Finally, the solution was centrifugated under the same conditions as above and after washing, resuspend in 100 µL of 10 mM phosphate buffer pH 7.4.

The average hydrodynamic size was measured with DLS (figure 6) before and after the conjugation, showing an increase of the size confirming a successful conjugation (table 1) (14).

**Table 1. Hydrodynamic diameter of SNPs in PLGA-PVA/PC before and after conjugation with neutravidin.**

| Sample | Size (nm) | Pdl |
|---|---|---|
| Before conjugation | 196 ± 3 | 0.166 |
| After conjugation | 263 ± 8 | 0.073 |

### Example 6: Lateral flow immunoassays

### 6.1. Preparation of the strips

The LFIA was based on a dipstick format. The test strips consist of a sample pad, a nitrocellulose membrane, an absorbent pad, and a backing plastic card. First, the 25 mm-wide-nitrocellulose membranes (CN95, Sartorius, Germany, FF120HP, Cytiva, Germany) was attached to a backing plastic card to obtain a robust system. Then, a test line of biotin-BSA was immobilized across the membrane by the IsoFlow dispenser (Imagene Technology, Lebanon, NH, USA) at a rate of 0.100 µL/mm with CN95 membranes and at 0.060 µL/mm with FF120HP membranes. After this, the membrane was dried for 20 min at 37 °C. Finally, the sample pad and the absorbent pad were stuck onto the backing card overlapping between them of 2 mm. The complete card was cut into 5 mm wide strips for the subsequent individual assays.

### 6.2. Lateral flow immunoassays assays (LFIA)

The use of starch nanoparticles as label in LFIAs were tested based in the reaction between the vitamin biotin and the protein neutravidin.

10 µL of the lipid polymer SNP-I-neutravidin conjugates were transferred into Eppendorf tubes with running buffer up to a final volume of 100 µL. The strips were added vertically and allowed to run for 15 min. After that, color intensity of the test line was quantified by reflectance measurements by using a portable strip reader ESE-Quant LR3 lateral flow system (Qiagen Inc., Hilden, Germany).

The signal obtained was then compared to another standard assay based also in Neutravidin/biotin LFIA but using 40 nm colloidal gold-neutravidin conjugates as a visual label.

Only the neutravidin coated nanoparticles were retained on the biotin-BSA test line. A simplified schematic representation of the biotin-neutravidin interaction in LFIA is shown in Figure 7.

The dried test line was analyzed by reflectance measurements (table 2). The results of the reflectance measurements for gold and hybrid nanoparticles were compared in terms of intensity (mV), with the density of the latter being slightly higher for a similar concentration of neutravidin, making this label a possible competitor for the gold nanoparticles.

**Table 2. Comparison of the intensity profiles measured for the gold nanoparticles and for the stained SNPs in the PLGA-PVA/PC nanoparticles.**

| Label | X-Position (mm) | Intensity (mV) | Peak start (mm) | Peak end (mm) | Height (mV) | Area (mm x mV) |
|---|---|---|---|---|---|---|
| SNPs-iodine in the PLGA-PVA/PC NPs | 20.8 | 932 | 19.76 | 21.76 | 208.27 | 173 |
| Au NPs | 20.8 | 809 | 19.96 | 21.96 | 237.18 | 239 |

### 6.3. Electrochemical measurement

The iodine and triiodide present in the starch nanoparticles in the test line was measured through coulometry. Nitrocellulose membranes with different surfactant treatments were used. After dried, test line was cut with a size of 4 mm × 4 mm, long enough to cover the working electrode. 2 µl of H₂SO₄ 0.5 M were added to the strip and overlayed to the SPCE. The auxiliary and reference electrode were not in contact with the membrane so another addition of 2 µl for each electrode was done to cover the entire electrochemical cell. Measurements were done by coulometry by applying a potential of 0.75 V for 900 s.

Next step was the measuring of hybrid nanoparticles in the test line after a lateral flow assay. Both types of membranes previously mentioned were tested. Results showed an increasing of charge in presence of nanoparticles in both types of membranes as it can be seen in Figure 8.

### Example 7: Electrochemical LFIA based on gold nanoparticles blocked with iodine-doped starch

### 7.1 Iodine staining of starch

A solution of native quinoa starch (reportedly with a 20.95% of amylose and a 79.05% of amylopectin14) in milliQ water was heated at 80°C for 30 min. Then, 2 mL of a solution of I2 5% and KI 10% were added, followed by the addition of 7 mL of absolute ethanol to precipitate the stained starch. The sample was then centrifuged for 4 min at 15880 x g. Finally, the supernatant was discarded, and the pellet was resuspended in milliQ water.

### 7.2 Bioconjugation of Au NPs

A solution of 1.5 mL of Au NPs with a 40 nm size was mixed with 100 µL of a neutravidin solution at different concentrations (0.15 and 0.3 mg/mL). After 1 h of reaction, 100 µL of a blocking solution containing native quinoa starch 0.1% stained with iodine were added. The mixture was incubated for 45 minutes with constant agitation. Then the nanoparticles were centrifuged for 20 min at 9184 × g and, after discarding the supernatant, they were resuspended in 2 mM phosphate stabilizing buffer at pH 7.4 with a concentration of 1% BSA and 10% sucrose. For comparison purposes, the same procedure was followed using 0.1% BSA as blocking agent.

### 7.3 Characterization of nanoparticle conjugates

### 7.3.1. Particle size distribution and ζ potential

The hydrodynamic size and homogeneity of the Au NPs and conjugates were determined by dynamic light scattering (DLS) using a Zetasizer Nano ZS instrument (Malvern Instruments Ltd., Malvern, UK). Samples were measured using the 173° backscatter detector in low volume disposable cuvettes (Malvern Instruments Ltd., Malvern, UK). The zeta potential was also measured using this technique in single use folded capillary cells (DTS1070).

The hydrodynamic size and the zeta-potential of the AuNP conjugates blocked with starch and BSA was determined by DLS measurements were carried out to confirm bioconjugation by comparing the size of the hydrodynamic radius before and after the conjugation reaction (Fig. 10). Table 3 summarizes the results obtained for each condition.

**Table 3: DLS measurements (ζ average, Pdl and ζ-potential) of the bare Au NPs and the conjugates blocked with BSA and iodine-doped starch.**

| | ζ average (nm) | Pdl | ζ-potential (mV) |
|---|---|---|---|
| Commercial Au NPs | 45 ± 1 | 0.157 | -32.2 ± 0.9 |
| Au-neutravidin blocked with BSA conjugate | 76 ± 2 | 0.182 | -13 ± 2 |
| Au-neutravidin blocked with iodine-doped starch | 75.0 ± 0.5 | 0.300 | -9 ± 3 |

The difference in size among the bare commercial nanoparticles and both conjugates proves that the conjugation process was successful in both cases. The main differences found between the conditions were found in the case of Pdl, analysis although the conjugates with the different blocks have similar sizes, in the case of the AuNPs blocked with starch-iodine the particles are slightly more polydisperse. This corresponds with what was observed in the ζ-potential measurements.

### 7.3.2. UV-Vis

The ultraviolet-visible spectrum was also measured to verify both conjugation and agglomeration of the nanoparticles. For this purpose, the absorption spectra of both the nanoparticles and the conjugates were measured on a Cary 60 UV-Vis spectrophotometer from Agilent Technologies (Palo Alto, CA, USA).

The absorbance measurements of gold nanoparticles were employed to track the adsorption of proteins and antibodies on their surface. A strong absorbance peak between 460-560 nm was observed due to the surface plasmon resonance (SPR) of colloidal gold, and its maximum wavelength (λₘₐₓ) varied depending on the size of the nanoparticles. The UV-Vis absorbance spectra of both the bare nanoparticles and the conjugates were measured between 350 nm and 800 nm (Fig. 11).

The maximum wavelength of AuNPs was 530 nm. A red-shift to a λₘₐₓ of 532 nm and 534 nm was observed when the conjugation reaction was performed, blocking with BSA and starch-iodine, respectively. This is an indication of a change in the refractive index of the nanoparticles due to the neutravidin layer on the surface of the nanoparticles.

### 7.4 Preparation of the strips

The LFIA was carried out based on a dipstick format. The test strips consisted of a sample pad, a nitrocellulose membrane (CN95, Sartorius, Germany), an absorbent pad, and a plastic backing card. First, the 25 mm wide nitrocellulose membranes were attached to a plastic backing card to obtain a robust system. A test line of biotin-BSA was then immobilized across the membrane using an IsoFlow dispenser (Imagene Technology, Lebanon, NH, USA) at a rate of 0.100 µL/mm. The membrane was then dried at 37°C for 30 minutes. Finally, the sample and absorbent pads were adhered to the backing card with an overlap of 2 mm. The complete card was cut into 5 mm wide strips for the subsequent individual assays.

### 7.5 Lateral flow immunoassay with electrochemical measurement

The iodine-doped starch-blocked nanoparticles were tested as labels in LFIAs based on the biotin-neutravidin affinity assay. The assay buffer was prepared by adding 10 µL of the gold nanoparticles conjugated (either the iodine-doped starch- or the BSA-blocked) to microcentrifuge tubes with running buffer, containing BSA, Tween 20 and NaCl up to a final volume of 100 µL. The strips were added vertically and allowed to run for 15 minutes.

At the end of the test, the test line (TL) was cut and put in an Eppendorf tube with 300 µL of PBST (0.01 M + 1% Tween-20) for 2 min to clean it from unspecific adsorptions. Then it was dried with paper and prepared to the screen-printed platform. Next step was the addition of 3µL of sulfuric acid 0.5 M to the membrane as an inert electrolyte. Then, it was coupled to the electrode and two additions of 2 µl of sulfuric acid were done to the auxiliary and reference electrode respectively. Finally, the electrochemical measurement was done by coulometry by applying a potential of +0.5 V for 100 s.

Only the neutravidin-coated nanoparticles were retained on the biotin test line. The test line was then left to dry, cut, to a stipulated size to cover the working electrode (4 mm), and handled as described above. Figure 12 shows the results of the coulometry for conjugates blocked with BSA. No significant trend in the measurements was observed as the analytical signal of the negative test is higher than the positive ones, which do not follow any trend either.

However, in the case of the conjugates blocked with iodine-doped starch (Fig. 13), a correlation between the concentration of neutravidin and the charge was obtained suggesting that by simply changing the blocking of the conjugates, electrochemical measurements can be performed, obtaining an electrochemical quantification procedure by using a standard LFIA based on Au NPs and overcoming one of its main limitations.

### List of References:

(1) Cheng, J.; Yang, G.; Guo, J.; Liu, S.; Guo, J. Integrated Electrochemical Lateral Flow Immunoassays (ELFIAs): Recent Advances. Analyst 2022, 147 (4), 554-570. https://doi.org/10.1039/D1AN01478A.
(2) Zou, Z.-X.; Wang, J.; Wang, H.; Li, Y.-Q.; Lin, Y. An Integrated Electrochemical Device Based on Immunochromatographic Test Strip and Enzyme Labels for Sensitive Detection of Disease-Related Biomarkers. Talanta 2012, 94, 58-64. https://doi.org/10.1016/j.talanta.2012.02.046.
(3) Liu, G.; Lin, Y.-Y.; Wang, J.; Wu, H.; Wai, C. M.; Lin, Y. Disposable Electrochemical Immunosensor Diagnosis Device Based on Nanoparticle Probe and Immunochromatographic Strip. Anal Chem 2007, 79 (20), 7644-7653. https://doi.org/10.1021/ac070691i.
(4) Wang, Y.; Wang, L.; Wang, S.; Yang, M.; Cai, J.; Liu, F. 'Green' Immunochromatographic Electrochemical Biosensor for Mercury(II). Microchimica Acta 2016, 183 (9), 2509-2516. https://doi.org/10.1007/s00604-016-1866-8.
(5) Mao, X.; Baloda, M.; Gurung, A. S.; Lin, Y.; Liu, G. Multiplex Electrochemical Immunoassay Using Gold Nanoparticle Probes and Immunochromatographic Strips. Electrochem commun 2008, 10 (10), 1636-1640. http s: Hdoi. org/ 10. 10 1 6/j. el ecom. 200 8.0 8.03 2.
(6) Zhu, X.; Shah, P.; Stoff, S.; Liu, H.; Li, C. A Paper Electrode Integrated Lateral Flow Immunosensor for Quantitative Analysis of Oxidative Stress Induced DNA Damage. Analyst 2014, 139 (11), 2850-2857. https://doi.org/10.1039/C4AN00313F.
(7) Thobhani, S.; Attree, S.; Boyd, R.; Kumarswami, N.; Noble, J.; Szymanski, M.; Porter, R. A. Bioconjugation and Characterisation of Gold Colloid-Labelled Proteins. J Immunol Methods 2010, 356 (1-2), 60-69. https://doi.org/10.1016/j.jim.2010.02.007.
(8) Gutiérrez, G.; Morán, D.; Marefati, A.; Purhagen, J.; Rayner, M.; Matos, M. Synthesis of Controlled Size Starch Nanoparticles (SNPs). Carbohydr Polym 2020, 250, 116938. https://doi.org/10.1016/j.carbpol.2020.116938.
(9) Padilla Mercado, J. B.; Coombs, E. M.; De Jesus, J. P.; Bretz, S. L.; Danielson, N. D. Iodine Coulometry of Various Reducing Agents Including Thiols with Online Photocell Detection Coupled to a Multifunctional Chemical Analysis Station To Eliminate Student End Point Detection by Eye. J Chem Educ 2018, 95 (5). https://doi.org/10.1021/acs.jchemed.7b00445.
(10) Bazsefidpar, S.; Moyano, A.; Gutiérrez, G.; Matos, M.; Blanco-López, M. C. Lipid-Polymer Hybrids Encapsulating Iron-Oxide Nanoparticles as a Label for Lateral Flow Immunoassays. Biosensors (Basel) 2021, 11 (7), 218. https://doi.org/10.3390/bios11070218.

## Claims

1. A nanoparticle for immunodetection based on lateral flow assay, wherein the nanoparticle comprises an iodine-starch complex.

2. The nanoparticle of claim 1, wherein the nanoparticle has a core and a shell, and the iodine-starch complex is located in the core of the nanoparticle.

3. The nanoparticle of claims 1 or 2, wherein the core of the nanoparticle comprises between about 1% - 4% (w/v), preferably between about 1% - 2% (w/v) of the iodine-starch complex.

4. The nanoparticle of claims 2 or 3, wherein the shell comprises at least one polyelectrolyte, at least one surface active agent and at least one stabilizer.

5. The nanoparticle of claim 4, wherein
(i) the polyelectrolyte is selected from the group consisting of polylactic-co-glycolic acid (PLGA), polylactic acid (PLA), poly-ε-caprolactone (PCL), or combinations thereof; and/or
(ii) the at least one surface-active is selected from phospholipids or non-ionic surfactants, or a combination thereof.

6. The nanoparticle of claim 5, wherein
(i) the phospholipid is selected from phosphatidylcholine, phosphatidylserine, or a combination thereof; and/or
(ii) the non-ionic surfactant is selected from Span 60, Span 80, or a combination thereof.

7. The nanoparticle of any one of claims 4 to 6, wherein the at least one stabilizer is selected from the group consisting of polyvinyl alcohol, dodecanol, cholesterol, cholesteryl hemi succinate, or combinations thereof.

8. The nanoparticle of any one of claims 1 to 7, wherein the mean diameter of the nanoparticle is between about 150 nm to about 250 nm, preferably between about 190 nm to about 210 nm.

9. The nanoparticle of claim 1, wherein the iodine-starch complex is located on the surface of the nanoparticle.

10. The nanoparticle of claim 9, wherein the nanoparticle is a gold nanoparticle (AuNP).

11. The nanoparticle of claims 9 or 10, wherein the nanoparticle has been blocked in a 0.1% starch (w/v) starch-iodine solution.

12. The nanoparticle of any one of claims 9 to 11, wherein the mean diameter of the nanoparticle including the iodine-starch complex on the surface is between about 60 nm to about 90 nm, preferably about 70 nm to about 80 nm.

13. A composition comprising a plurality of nanoparticles of any one of the preceding claims.

14. An immunodetection sensor comprising the nanoparticle for immunodetection based on lateral flow assay or the composition comprising a plurality of nanoparticles according to any one of the preceding claims.

15. An immunodetection method comprising the steps of
(i) mixing a sample with the nanoparticle(s) according to any one of claims 1 to 12 or the composition comprising a plurality of nanoparticles according to claim 13, wherein the nanoparticle(s) have been conjugated to the antibody or protein for detection of an analyte in a sample; and
(ii) detecting an electrochemical signal, an optical signal, or both.
